Europäisches Patentamt

European Patent Office

Office européen des brevets

(11) Veröffentlichungsnummer: **0 224 641**

**A1**

## (12) EUROPÄISCHE PATENTANMELDUNG

(21) Anmeldenummer: 86109076.9

(51) Int. Cl.⁴ **A61M 5/31** , A61M 5/14

(22) Anmeldetag: 03.07.86

(30) Priorität: 09.10.85 DE 8528780 U

(43) Veröffentlichungstag der Anmeldung:
10.06.87 Patentblatt 87/24

(84) Benannte Vertragsstaaten:
AT BE CH DE FR GB IT LI LU NL SE

(71) Anmelder: **INTERMEDICAT GMBH**
**Gerliswilstrasse 74**
**CH-6020 Emmenbrücke(CH)**

(72) Erfinder: **Herlitze, Gerhard**
**Binsdorfer Strasse 4**
**D-3507 Baunatal 7(DE)**

(74) Vertreter: **von Kreisler, Alek, Dipl.-Chem. et al**
**Deichmannhaus am Hauptbahnhof**
**D-5000 Köln 1(DE)**

(54) **Injektionsventil.**

(57) Die Erfindung betrifft ein Injektionsventil für eine Infusionseinrichtung mit einem einen kanalartigen Hohlraum (12) enthaltenden Gehäusekörper (11), der einen den Hohlraum (12) mit einer zu einem Patienten führenden Zuleitung verbindenden Auslaß aufweist und in dessen Wand sich eine Öffnung (19) befindet, die den Hohlraum (12) mit einem zu seiner Längsachse quergerichteten Anschlußstutzen (20) für den Außenkonus einer Spritze verbindet und mit einem in dem Hohlraum (12) koaxial angeordnetem Ventilschlauch (15), dessen Außenfläche gegen die Öffnung (19) anliegt und von der Wand des Hohlraumes (12) radial wegdrückbar ist. Sie ist dadurch gekennzeichnet, daß die Stirnfläche (15a) des patientenseitigen Randes des Ventilschlauches (15) gegen eine Ringschulter (17) in der Wand (16) des Hohlraumes (12) anliegt. Auf diese Weise wird ein Injektionsventil mit einem leichtgängig betätigbaren Ventilschlauch sicherer gestaltet.

FIG. 1

# Injektionsventil

Die Erfindung bezieht sich auf ein Injektionsventil für eine Infusionseinrichtung mit einem einen kanalartigen Hohlraum enthaltenden Gehäusekörper, der einen den Hohlraum mit einer zu einem Patienten führenden Zuleitung verbindenden Auslaß aufweist und in dessen Wand sich eine Öffnung befindet, die den Hohlraum mit einem zu seiner Längsachse quergerichteten Anschlußstutzen für den Außenkonus einer Spritze verbindet und mit einem in dem Hohlraum koaxial angeordneten Ventilschlauch, dessen Außenfläche gegen die Öffnung anliegt und von der Wand des Hohlraumes radial wegdrückbar ist.

Injektabilien, wie Antibiotika, Vitamine usw. werden in Spritzen aus Ampullen aufgezogen und dem Patienten unmittelbar entweder intravenös oder intramuskulär mittels einer auf den Spritzenaußenkonus aufgesetzten Kanüle verabreicht. Wurde bei einem Patienten bereits ein Venenzugang entweder durch einen Kurzkatheter im Handrücken bzw. Unterarmbereich oder durch einen Katheter in großlumigen Gefäßen geschaffen, so besteht die Möglichkeit, Injektabilien durch Ankopplung des Außenkonus der Spritze ohne angesetzte Stahlkanüle an den Katheteransatz oder an ein in eine Infusionsleitung integriertes Injektionszwischenstück zu verabreichen. Sowohl bei dem Katheteransatz als auch bei dem Injektionszwischenstück ist der Gehäusekörper mit einem zur Längsachse des Hohlraumes quergerichteten Anschlußstutzen für den Außenkonus der Spritze versehen und die Öffnung zur Verbindung des Anschlußstutzens mit dem Hohlraum ist durch ein Ventilelement abgedichtet. Es sind unterschiedliche Ventilelemente bekannt. Sie können z.B. als elastisch niederdrückbarer Verschlußstopfen (DE-PS 31 32 323), als eindrückbarer, geschlitzter, elastischer Bereich der Wand des Hohlraums (DE-PS 33 03 073) oder als radial wegdrückbarer Ventilschlauch - (DE-PS 12 16 489) ausgebildet sein.

Bei den beiden erstgenannten Ventilelementarten ist nachteilig, daß diese durch Andrücken des Spritzenkegels gegen das Ventilelement geöffnet werden müssen und daß sich bei Verwendung der Injektionsventile in Venenverweilkanülen der Druck als Flächenpressung auf den Patienten auswirkt und diesen belastet. Leichtgängiger ist ein Ventilschlauch, der sich unter dem Druck des Injektats wegbiegt und die Öffnung freigibt. Bei dem bekannten Injektionsventil (DE-PS 12 16 489) ist ein in den Hohlraum koaxial hineinragendes Einsatzstück mit einer als Ventilkörper dienenden hülsenförmigen Verlängerung in Form eines Kunststoffschlauches ausgebildet, der gegen die Öffnung abdichtend anliegt. Der im Bereich des Kunststoffschlauches zylindrische Hohlraum geht patientenseitig stufenlos in eine konische Mischungskammer über und an der Grenze zwischen beiden Abschnitten liegt lose das freie ungehaltene Ende des Kunststoffschlauches. Es besteht die Gefahr, daß insbesondere bei mehrmaliger Betätigung des Injektionsventils die Befestigung des Kunststoffschlauches an dem Einsatzstück sich löst und der Kunststoffschlauch sich in Richtung der konischen Mischungskammer verschiebt. Dann ist die Öffnung in der Wand des Gehäusekörpers nicht mehr ordnungsgemäß abgedichtet und Luftblasen und Kontaminationen können mit dem Infusionsstrom in den Patienten gelangen. Außerdem ist bei vollständigem Abreißen des Ventilschlauches von dem Einsatzstück zu befürchten, daß der Ventilschlauch so weit in die konische Mischungskammer vordringt, daß sein lichter Querschnitt radial vollständig zusammengepreßt und der Durchströmungskanal verstopft wird.

Der Erfindung liegt die Aufgabe zugrunde, ein Injektionsventil mit einem leichtgängig betätigbaren Ventilschlauch sicherer zu gestalten.

Diese Aufgabe wird erfindungsgemäß dadurch gelöst, daß die Stirnfläche des patientenseitigen Randes des Ventilschlauches gegen eine Ringschulter in der Wand des Hohlraumes anliegt.

Durch die Abstützung des einen Endes des Ventilschlauches an der Gehäuse-Ringschulter ist der Ventilschlauch gegen eine Verschiebung in Richtung des patientenseitigen Endes sicher gehalten, und zwar sowohl bei geschlossenem als auch bei geöffnetem Injektionsventil. Der wandschlüssige Ventilschlauch liegt spannungsfrei in dem Hohlraum, weil er mit der Hohlraumwand nicht verbunden ist. Hierdurch wird die zuverlässige Abdichtung der Öffnung zwischen Anschlußstutzen und Hohlraum garantiert, und bei radialer Druckausübung gegen den Ventilschlauch infolge des Einspritzens von Injektat wird ein Teil des Ventilschlauchumfanges flexibel ausgelenkt und gibt den Weg durch die Öffnung frei, während ein anderer Teil des Ventilschlauchumfanges immer an der Ringschulter abgestützt bleibt und als Halterung wirksam ist. Auch bei wiederholtem Zuspritzen von Flüssigkeiten durch den Anschlußstutzen ist der dichte Verschluß der Öffnung durch die spannungsfreie Lagerung des Ventilschlauches gewährleistet. Der Ventilschlauch kann durch seine erfindungsgemäße Verschiebungssicherung ohne Beeinträchtigung der Öffnungs-und Schließwirkung verhältnismäßig lang ausgebildet sein, so daß er der Öffnung nach beiden Seiten weit überragt. Da keine Befestigung des Ventilschlauches z.B. durch Verklebung oder Einklemmen erfolgt, ist sein Montage einfach. Er wird axial bis zum Anschlag gegen die Ringschulter in den Hohlraum eingeschoben. Zum Verschluß des Hohlraumes des Gehäusekörpers an dem patientenfernen Ende wird auf dieses Ende nach dem Einführen des Ventilschlauches lediglich eine Verschlußkappe aufgesetzt und der den Hohlraum mit einer zu einem Patienten führenden Zuleitung verbindende Auslaß ist nur mit dem Anschlußstutzen für den Außenkonus der Spritze verbindbar. Wenn jedoch der kanalartige Hohlraum an beiden Enden offen ist, so daß ein Durchgangskanal entsteht, ist der den Hohlraum mit einer zu einem Patienten führenden Zuleitung verbindende Auslaß auch an einen Infusionsflüssigkeitsbehälter anschließbar und es kann dem Infusionsstrom durch das Injektionsventil beliebig oft eine ergänzende Injektion von Arzneimitteln mit Hilfe einer Spritze zugefügt werden. Die Leichtgängigkeit der Auslenkung des Ventilschlauches, seine elastische Rückstellfähigkeit und seine Verschiebungssicherung mit Hilfe der Ringschulter ermöglichen allerdings auch eine längere Infusion aus einem Infusionsflüssigkeitsbehälter durch den Anschlußstutzen. In diesem Falle wird der Anschlußstutzen für den Außenkonus einer Spritze mit einem Außenkonus-Verbindungsstück einer zu dem Infusionsflüssigkeitsbehälter führenden Leitung verbunden.

In vorteilhafter Ausgestaltung der Erfindung ist die Ringschulter auf einem Teil ihres Umfangs ausgespart. Es kann eine einzige Aussparung in dem der Öffnung zugewandten Umfangsbereich des Hohlraumes vorgesehen sein oder es können mehrere Aussparungen über den Umfang der Ringschulter verteilt sein. Die mindestens eine Aussparung an der Ringschulter erleichtert die Öffnung des Injektionsventils, weil es zum Durchlaß der Flüssigkeit nicht erforderlich ist, daß die Stirnfläche des patientenseitigen Randes des Ventilschlauches an irgendeiner Stelle vollständig von der Ringschulter radial weggedrückt wird. Ein sehr geringfügiges Wegdrücken des Ventilschlauches von der Wand des Hohlraumes genügt, da die Flüssigkeit durch die mindestens eine Aussparung der Ringschulter in den Hohlraum eintreten kann. Auf diese Weise genügen auch sehr kleine Zuspritzmengen für die zufriedenstellende Betätigung des Injektionsventils. Es kann vorgesehen sein, daß die Aussparung sich umfangsmäßig über einen Winkelbereich von etwa 90°, vorzugsweise etwa 180°, erstreckt. In diesem Falle befindet sich die eine Aussparung in dem der Wandöffnung an dem Anschlußstutzen zugewandten Umfangsbereich des Hohlraumes. Bei mehreren Aussparungen können diese einen größeren Umfangsbereich einnehmen als die Ringschulter, so daß die Ringschulter praktisch auf einige radiale Rippen reduziert ist, gegen die die Stirnfläche des patientenseitigen Randes des Ventilschlauches anliegt.

Erfindungsgemäß kann vorgesehen sein, daß der kanalartige Hohlraum an beiden Enden offen ist und daß der Gehäusekörper als Injektionszwischenstück ausgebildet ist, das an beiden Enden je einen Verbindungteil zum Anschluß eines Leitungs-Kupplungselementes aufweist. In diesem Falle gehört das Injektionsventil zu einem Injektionszwischenstück, das in eine Infusionsleitung einsetzbar ist. Der Aufbau des Injektionsventils aus nur wenigen Teilen trägt dazu bei, daß das Injektionszwischenstück sehr leicht ist und die Infusionsleitung nicht beschwert. Die leichtgängige Auslenkbarkeit des Ventilschlauches verhindert Abknickungen der Infusionsleitung beim Zuspritzen von Medikamenten durch Handhabungsschwierigkeiten.

Alternativ kann bei an beiden Enden offenem kanalartigem Hohlraum vorgesehen sein, daß der Gehäusekörper als Katheteransatz ausgebildet ist, der an einem Ende einen Verbindungteil zum Anschluß eines Leitungs-Kupplungsteiles aufweist und an dessen anderem Ende ein Katheter (flexibel) oder eine Kanüle (starr) befestigt ist.

Die Stirnfläche des patientenfernen Randes des Ventilschlauches kann abstützungslos sein, so daß sie bei in dem Gehäusekörper ausgebildetem Durchgangskanal dem Injektionsflüssigkeitsstrom frei entgegengerichtet ist und von diesem in Strömungsrichtung so belastet wird, daß er die Stirnfläche des patientenseitigen Randes des Ventilschlauches gegen die vordere Ringschulter preßt

Vorteilhafterweise ist auf das patientenferne Ende des Gehäusekörpers eine Ringkappe aufgesteckt, die außen den einen Verbindungteil aufweist und innen mit einer Ringschulter zur Abstützung der Stirnfläche des patientenfernen Randes des Ventilschlauches versehen ist. Die Verbindungteile an dem Gehäusekörper können beliebig ausgebildet und den anzuschließenden Leitungs-Kupplungselementen jeweils angepaßt sein.

In der Zeichnung sind zwei Ausführungsbeispiele der Erfindung schematisch dargestellt.

Die beiden Figuren zeigen im Längsschnitt zwei verschiedene Ausführungsformen von Injektionszwischenstücken zum Einbau in eine Infusionsleitung.

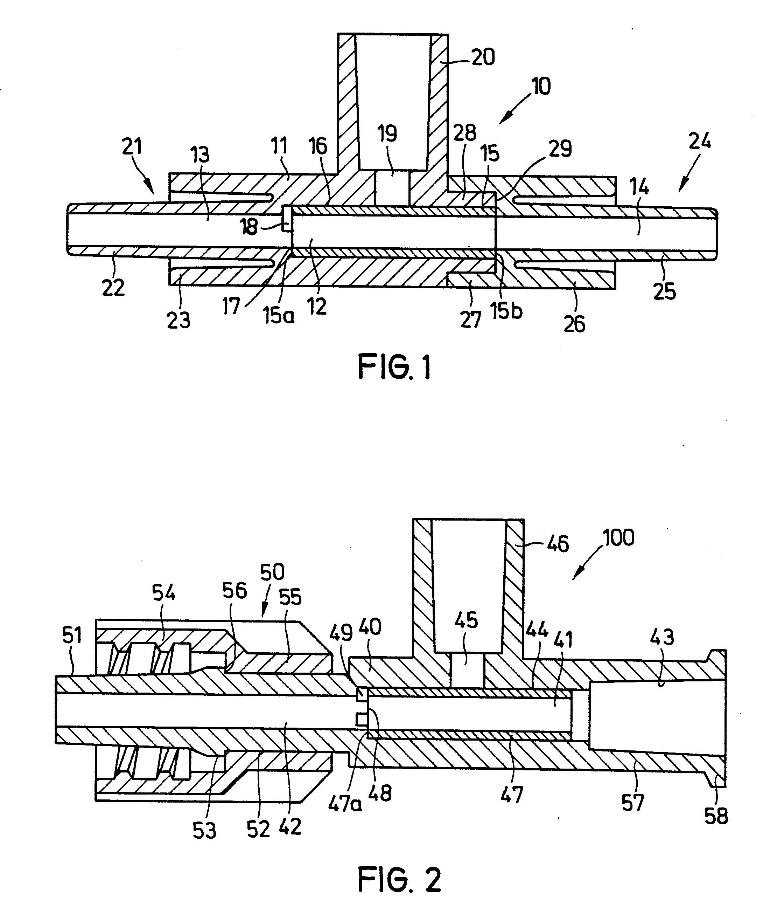

Gemäß Fig. 1 besteht ein selbständiges Injektionszwischenstück 10 aus einem Gehäusekörper 11, der als gerade Hülse mit einem geraden, kreiszylindrischen Hohlraum 12 ausgebildet ist, dessen offene Enden koaxialen, ebenfalls kreiszylindrischen Verlängerungen 13,14 fortgesetzt werden. Der Durchmesser des Hohlraumes 12 ist in bezug auf die Durchmesser der identischen Verlängerungen 13, 14 um die Wandstärke eines sehr flexiblen, hochgradig elastisch nachgiebigen Ventilschlauches 15 größer, der in den Hohlraum 12 koaxial eingelegt ist. Der Ventilschlauch 15 liegt stramm gegen die zylindrische Wand 16 des Hohlraumes 12 an, ohne an irgendeiner Stelle an dieser befestigt zu sein. Der Ventilschlauch 15 ist an seinen beiden Enden gerade, d.h. rechtwinklig zu seiner Längsachse, abgeschnitten und hat stumpfe Stirnflächen 15a,15b. Das in der Zeichnung linke Ende des Gehäusekörpers 11 ist im Anwendungsfalle einem Patienten zugewandt und die gerade unterbrechungslose Stirnfläche 15a des patientenseitigen Randes des Ventilschlauches 15 stößt gegen eine rechtwinklige, zu der Stirnfläche 15a parallele Ringschulter 17 an, die sich durch die unterschiedlichen Durchmesser des Hohlraumes 12 und der Verlängerung 13 ergibt und deren Bemessung dazu führt, daß bei eingelegtem Ventilschlauch 15 der Durchgangskanal am Übergang zwischen dem Hohlraum 12 und der Verlängerung 13 stufenlos ist. Die Ringschulter 17 ist bei dem Beispiel der Fig. 1 über einen Winkelbereich von etwa 180° axial ausgespart. Auf diese Weise entsteht in der Flucht des Ventilschlauches 15 eine halbkreisförmige Aussparung 18 gleichmäßiger axialer Tiefe, die die entsprechende Zone der Stirnfläche 15a des Ventilschlauches 15 von der Ringschulter 17 freisetzt, so daß dieser Teilumfangsbereich der Stirnfläche 15a des Ventilschlauches 15 nicht axial abgestützt ist.

Etwa in der Quermitte des Hohlraumes 12 und des Ventilschlauches 15 befindet sich in der Wand des Gehäusekörpers 11 eine seitliche, durchgehende Öffnung 19, an die sich außen ein radialer Anschlußstutzen 20 anschließt, der zur Aufnahme des Außenkonus einer Spritze innen konisch gestaltet ist. Diese Öffnung 19 wird von der sie innen bedeckenden Außenflächenzone des Ventilschlauches 15 verschlossen, wenn dieser sich in der gezeigten entspannten Grundstellung befindet. Die Verschlußwirkung des Ventilschlauches 15 wird durch den radialen Druck der den Hohlraum 12 und seine Verlängerungen 13 und 14 durchströmenden Infusionsflüssigkeit erhöht, die den Ventilschlauch 15 radial nach außen preßt. Wenn der Infusionsflüssigkeit ergänzende flüssige Arzneimittel beigefügt werden sollen, werden diese in einer Spritze aufgezogen, die ohne Metallkanüle mit ihrem Außenkonus in den Anschlußstutzen 20 eingesetzt und betätigt wird. Die gegen die Außenfläche des Ventilschlauches 15 radial andrückende ausgespritzte Flüssigkeitsmenge verformt den Ventilschlauch 15 der art, daß sich ein Spalt zwischen Außenfläche des Ventilschlauches 15 und Wand 16 des Hohlraumes 12 ergibt, durch den die Flüssigkeit hindurchtritt. Da sie im Bereich der Aussparung 18 frei abströmt und sich nicht an der Ringschulter 17 vorbeizwängen muß, ist auch bei der Abgabe sehr geringer Flüssigkeitsmengen und entsprechend niedrigem Spritzendruck eine zur Öffnung des Injektionsventils ausreichende radiale Eindrückung des Ventilschlauches 15 gewährleistet. Die axiale Tiefe der Aussparung 18 kann gering sein. Beispielsweise kann sie etwa der Wandstärke des Ventilschlauches 15 entsprechen.

Die patientenseitige zylindrische Verlängerung 13 des Hohlraumes 12 kann sich in einem Verbindungsteil 21 befinden, der einen glatten Außenkonus 22 aufweist, welcher über einen Teil seiner axialen Länge mit Abstand von einem außen und innen glatten Mantel 23 umgeben ist. Der Mantel 23 überfängt das freie Ende eines der Deutlichkeit halber nicht gezeigten Leitungs-Kupplungselementes, das auf den Außenkonus 22 aufgesteckt ist und das Injektionszwischenstück 10 mit einer zu einem Patienten führenden Leitung verbindet. Der Verbindungsteil 24 am anderen Ende des Gehäusekörpers 11 ist ebenfalls mit einem glatten Außenkonus 25 und einem diesen auf einem Teil seiner Länge mit Abstand umgebenden außen und innen glatten Mantel 26 ausgestattet. Allerdings befinden sich diese Teile an einer Ringkappe 27, die auf einen angepaßten koaxialen Stutzen 28 des Gehäusekörpers 11 aufgesteckt und ggf. durch Klebung oder Schweißung befestigt ist. Der zur Längsachse des Gehäusekörpers 11 rechtwinklige, ebene Ringboden 29 der Ringkappe 27 überragt die rechtwinklige ebene Stirnfläche des Stutzens 28 radial um ein der Wandstärke des Ventilschlauches 15 etwa entsprechendes Stück, so daß die patientenferne Stirnfläche 15b des Ventilschlauches 15 sich an der von dem inneren radialen Überstand des Ringbodens 29 gebildeten Ringschulter der Ringkappe 27 abstützen kann und eine Verschiebung des an der Wand 16 des Hohlraumes 12 nicht befestigten Ventilschlauches 15 in Richtung des patientenfernen Endes verhindert. Sowohl die patientenseitige Stirnfläche 15a als auch die patientenferne Stirnfläche 15b sind auf diese Weise axial abgestützt.

Bei dem Beispiel gemäß Fig. 2 ist das erfindungsgemäße Injektionsventil ebenfalls bei einem Injektionszwischenstück 100 realisiert. Dieses besteht aus einem Gehäusekörper 40 mit kreiszylindrischem Hohlraum 41, der an dem linken patientenseitigen Ende von einer kreiszylindrischen Verlängerung 42 kleineren Durchmessers fortgesetzt wird, während sich an sein patientenfernes Ende ein Innenkonus 43 größeren Querschnittes anschließt. In der Wand des Gehäusekörpers 40 ist eine durchgehende seitliche Öffnung 45 ausgebildet, die einen radialen äußeren Anschlußstutzen 46

für einen Spritzenkonus mit dem Hohlraum 41 verbindet. Der Hohlraum 41 enthält einen krieszylindrischen Ventilschlauch 47, der umfangsmäßig stramm in den Hohlraum 41 eingepaßt ist und dessen patientenseitige Stirnfläche 47a sich an einer Ringschulter 48 in der Wand 44 des Hohlraumes 41 abstützt. Die Ringschulter 48 ist umfangsmäßig unterbrochen, so daß mehrere axiale Aussparungen 49 entstehen, die gleiche Tiefe und gleiche oder verschiedene Breite haben können. Die Stirnfläche 47a wird auf diese Weise auf den geraden Zinnenflächen der Ringschulter 48 abgestützt und durch die Zwischenräume zwischen den Zinnen kann Flüssigkeit in den Hohlraum 41 bzw. die Verlängerung 42 eindringen, wenn sie mit Hilfe einer Spritze durch die Öffnung 45 und zwischen dem Ventilschlauch 47 und der Wand 44 des Hohlraumes 41 hindurchgedrückt worden ist. Diese Passage ergibt sich dadurch, daß die zugespritzte Flüssigkeit das Ventilschlauchstück zwischen der Öffnung 45 und der Ring schulter 48, das mit der Wand 44 des Hohlraumes 41 nicht verbunden ist, radial nach innen drückt. Bei Beendigung der Zuspritzung wird der Ventilschlauch 47 von dem kontinuierlichen Infusionsflüssigkeitsstrom wieder radial nach außen gegen die Öffnung 45 gedrückt. Bei diesem Beispiel ist das patientenferne Ende des Ventilschlauches 47 gegen axiale Verschiebung nicht durch eine Ringschulter gesichert, und zur Verhinderung seines Herausfallens bei Nichtbenutzung des Injektionszwischenstückes 100 empfiehlt es sich, das Ventilschlauchstück . zwischen der Öffnung 45 und dem Innenkonus 43 mit der Innenwand des Hohlraumes 41 z.B. zu verkleben.

Die Verlängerung 42 des Hohlraumes 41 und der Innenkonus 43 sind in Verbindungsteilen ausgebildet, die an den Gehäusekörper 40 einstückig angeformt sind. Der patientenseitige Verbindungsteil 50 besteht aus einem Außenkonus 51 an einem zylindrischen Halsstück 52, das durch einen Bund 53 von dem Außenkonus 51 getrennt ist. Der Außenkonus 51 ist auf einem Teil seiner Länge von einer Innengewindekappe 54 umgeben, die eine Buchse 55 aufweist, welche das Halsstück 52 drehbar umgibt und mit einem Absatz 56 den Bund 53 hintergreift, so daß die Innengewindekappe 54 gegen axiales Abziehen gesichert ist.

Der Innenkonus 43 ist in einem Stutzen 57 ausgebildet, der an seinem äußeren Rand radial nach außen abstehende Verriegelungsnocken 58 aufweist, die mit dem Innengewinde einer Sicherungsmutter an einem Leitungs-Kupplungselement zusammengreifen.

Das erfindungsgemäße Injektionsventil läßt sich in ähnlicher Weise wie in den Fig. 1 und 2 gezeigt, auch bei einem Katheteransatz anwenden, der einen seitlich abgehenden Anschlußstutzen aufweist und an seinem patientenseitigen Ende mit einem längeren oder kürzeren Katheterschlauch bzw. einer Stahlkanüle verbunden ist.

## Ansprüche

1. Injektionsventil für eine Infusionseinrichtung mit einem einen kanalartigen Hohlraum enthaltenden Gehäusekörper, der einen den Hohlraum mit einer zu einem Patienten führenden Zuleitung verbindenden Auslaß aufweist und in dessen Wand sich eine Öffnung befindet, die den Hohlraum mit einem zu seiner Längsachse quergerichteten Anschlußstutzen für den Außenkonus einer Spritze verbindet und mit einem in dem Hohlraum koaxial angeordnetem Ventilschlauch, dessen Außenfläche gegen die Öffnung anliegt und von der Wand des Hohlraumes radial wegdrückbar ist,
**dadurch gekennzeichnet**, daß
die Stirnfläche (15a)des patientenseitigen Randes des Ventilschlauches (15) gegen eine Ringschulter (17) in der Wand (16) des Hohlraumes (12) anliegt.

2. Injektionsventil nach Anspruch 1,
**dadurch gekennzeichnet**, daß
die Ringschulter (17) auf einem Teil ihres Umfanges ausgespart ist.

3. Injektionsventil nach Anspruch 2,
**dadurch gekennzeichnet**, daß
eine Aussparung (18) in dem der Wandöffnung - (19) zugewandten Umfangsbereich des Hohlraumes (12) vorgesehen ist.

4. Injektionsventil nach Anspruch 3,
**dadurch gekennzeichnet**, daß
die Aussparung (18) sich umfangsmäßig über einen Winkelbereich von etwa 90°, vorzugsweise etwa 180°, erstreckt.

5. Injektionsventil nach Anspruch 2,
**dadurch gekennzeichnet**, daß
mehrere Aussparungen (49) über den Umfang der Ringschulter (48) verteilt sind.

6. Injektionsventil nach Anspruch 5,
**dadurch gekennzeichnet**, daß
die Aussparungen (49) einen größeren Umfangsbereich einnehmen als die Ringschulter (48).

7. Injektionsventil nach einem der Ansprüche 1 bis 6,
**dadurch gekennzeichnet**, daß
der kanalartige Hohlraum (12) an beiden Enden offen ist und daß der Gehäusekörper (11) als Injektionszwischenstück ausgebildet ist, das an beiden Enden je einen Verbindungsteil (21;24) zum Anschluß je eines Leitungs-Kupplungslementes aufweist.

8. Injektionsventil nach einem der Ansprüche 1 bis 6,

**dadurch gekennzeichnet,** daß der kanalartige Hohlraum an beiden Enden offen ist und daß der Gehäusekörper als Katheteransatz ausgebildet ist, der an einem Ende einen Verbindungsteil zum Anschluß eines Leitungs-Kupplungsteiles aufweist und an dessen anderem Ende ein Katheter oder eine Kanüle befestigt ist.

·9. Injektionsventil nach einem der Ansprüche 7 oder 8,

**dadurch gekennzeichnet,** daß auf das patientenferne Ende des Gehäusekörpers (11) eine Ringkappe (27) aufgesteckt ist, die außen den einen Verbindungsteil (24) aufweist und innen mit einer Ringschulter (29) zur Abstützung der Stirnfläche - (15b) des patientenfernen Randes des Ventilschlauches (15) versehen ist.

FIG. 1

FIG. 2

| | **EINSCHLÄGIGE DOKUMENTE** | | EP 86109076.9 |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl. 4) |
| Y,D | DE - B - 1 216 489 (SJUCC AKTIE-BOLAG) | 1,7,8 | A 61 M 5/31 |
| A | * Gesamt * | 9 | A 61 M 5/14 |
| | -- | | |
| Y | EP - A1 - 0 101 534 (INTERMEDICAT) | 1,7,8 | |
| | * Gesamt; insbesondere Fig. 1; Seite 6, Zeile 27 - Seite 7, Zeile 6 * | | |
| | -- | | |
| A | DE - A1 - 2 747 276 (VIGGO AB) | 1 | |
| | * Gesamt; insbesondere Fig. 5; Seite 5, letzter Absatz - Seite 6, 1. Absatz * | | |
| | -- | | |
| D,A | DE - C1 - 3 303 073 (B.BRAUN MELSUNGEN) | 1 | |
| | * Gesamt; insbesondere Fig. 1,2; Zusammenfassung * | | RECHERCHIERTE SACHGEBIETE (Int. Cl. 4) |
| | -- | | A 61 M 5/00 |
| A | DE - A1 - 3 002 120 (BECTON, DICKINSON) | 1 | A 61 M 25/00 |
| | * Gesamt; insbesondere Fig. 2A, 2B; Seite 13, 3. Absatz - Seite 16, 1. Absatz * | | |
| | -- | | |
| D,A | DE - A1 - 3 132 323 (B.BRAUN MELSUNGEN) | 1 | |
| | * Gesamt; insbesondere Fig. 4; Zusammenfassung * | | |
| | -- | | |

Der vorliegende Recherchenbericht wurde für alle Patentansprüche erstellt.

| Recherchenort | Abschlußdatum der Recherche | Prüfer |
|---|---|---|
| WIEN | 13-03-1987 | LUDWIG |

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| P,A | EP - A2 - 0 160 807 (INTERMEDICAT 13-11-1985)<br>* Gesamt; insbesondere Fig. 1,4; Seite 6, Zeile 1 - Seite 7, Zeile 14; Seite 8, Zeile 10 - Seite 9, Zeile 8 *<br><br>& DE-A1-3 417 257 (14-11-1985)<br>---- | 1 | |

RECHERCHIERTE SACHGEBIETE (Int. Cl.4)

Der vorliegende Recherchenbericht wurde für alle Patentansprüche erstellt.

| Recherchenort | Abschlußdatum der Recherche | Prüfer |
|---|---|---|
| WIEN | 13-03-1987 | LUDWIG |